(19) Europäisches Patentamt

European Patent Office

Office européen des brevets

(11) Numéro de publication : **0 536 022 A1**

(12) **DEMANDE DE BREVET EUROPEEN**

(21) Numéro de dépôt : 92402616.4

(22) Date de dépôt : 24.09.92

(51) Int. Cl.$^5$ : **C12M 1/34,** C12Q 1/18, C12M 3/06

(30) Priorité : 01.10.91 FR 9112036

(43) Date de publication de la demande :
07.04.93 Bulletin 93/14

(84) Etats contractants désignés :
AT BE CH DE DK ES FR GB GR IE IT LI LU MC NL PT SE

(71) Demandeur : **Société Anonyme dite : BIOGIR Zone Industrielle de Toctoucau F-33610 Cestas (FR)**

(72) Inventeur : **Boue-Grabot, Marc**
**56, rue des Menuts**
**F-33000 Bordeaux (FR)**
Inventeur : **Pinon, Jean-François**
**77, avenue du Maréchal Leclerc**
**F-33400 Talence (FR)**
Inventeur : **Saboureau, Dominique**
**27, avenue de la Gare**
**F-33610 Gazinet-Cestas (FR)**

(74) Mandataire : **Lepeudry-Gautherat, Thérèse et al**
**Armengaud Jeune Cabinet Lepeudry 52, avenue Daumesnil**
**F-75012 Paris (FR)**

(54) **Matériel prêt à l'emploi permettant la réalisation d'études de toxicologie et de pharmacologie sur des cellules en culture.**

(57) L'invention concerne un matériel prêt à l'emploi permettant des études de toxicologie et de pharmacologie sur des cellules conservées dans des chambres de cultures individuelles intégrées dans un bloc de plastique permettant à la fois leur stockage, leur transport et leur utilisation expérimentale, grâce à l'utilisation d'un milieu de maintenance gélifié à basse température.

L'invention est destinée par exemple aux laboratoires de produits cosmétiques ne pouvant pas s'équiper pour réaliser des cultures de cellules.

L'invention représente une alternative aux tests de toxicité sur animaux.

EP 0 536 022 A1

Jouve, 18, rue Saint-Denis, 75001 PARIS

La présente invention a pour objet un matériel prêt à l'emploi permettant la réalisation d'études de toxicologie et de pharmacologie sur des cellules en culture. Le matériel selon l'invention comporte un nombre limité de chambres de culture permettant l'établissement des cellules sur une membrane microporeuse qui assure le contact des cellules avec les substances à tester. Le matériel peut être stocké et transporté à basse température puis remis à incuber pour être utilisé, sans autre manipulation, dans des tests de toxicité vis à vis des cellules.

De nombreux efforts sont entrepris par les laboratoires pharmaceutiques et cosmétiques pour développer des systèmes alternatifs afin de limiter le nombre de tests de toxicité sur animaux. Plusieurs tests s'effectuent déjà sur des "équivalents de tissus", sur des fragments d'organes en culture ou des monocouches cellulaires. Plusieurs laboratoires proposent déjà à la vente différents types de cellules, à l'état congelé, pour la mise en culture et l'application en toxicologie. Toutefois ces cultures ne peuvent être réalisées que dans des laboratoires équipés en matériel sophistiqué et en personnel bien entraîné aux cultures de cellules.

La mise à disposition de matériel prêt à l'emploi représenterait donc un avantage pour de nombreux laboratoires qui ne peuvent s'équiper pour réaliser eux mêmes des cultures cellulaires.

Le matériel selon l'invention comprend un support de culture constitué d'un moulage en matière plastique en deux parties ajustées :

- une partie a) constituée d'un monobloc de matière plastique non toxique pour les cellules supérieures, de forme quelconque, cylindrique ou parallélipipédique par exemple, dans lequel sont creusés des puits cylindriques, servant de "chambre de culture" pour des cellules supérieures en monocouche ; la partie inférieure des puits est constituée d'une membrane microporeuse en matière compatible avec l'attachement et la multiplication des cellules, ladite membrane étant par exemple thermosoudée au bloc de plastique ; la partie supérieure des puits est obturée, à l'exception des orifices de remplissage qui seront fermés ultérieurement par un film de plastique adhésif ;
- une partie b) de préférence moulée dans la même matière que le bloc a) et formant un support inférieur adapté pour fermer hermétiquement le bloc a) tout en ménageant un espace libre entre les membranes microporeuse et le fond du support ; cet espace libre sera rempli de milieu de culture ou du milieu de maintenance des cellules ; il pourra être commun à tous les puits ou être compartimenté pour permettre ultérieurement le test de différents produits ou de différentes doses de produit.

Selon un mode particulier de réalisation, la partie a) est allégée et se présente sous forme d'une plaque conçue pour s'ajuster sur la partie b), ladite plaque étant munie du côté inférieur, de saillies formant puits de cultures.

Quand les deux parties a) et b) sont emboîtées, l'ensemble est de préférence fermé par un film de plastique qui lui assure une parfaite étanchéité, et recouvert d'un couvercle protecteur permettant la superposition de plusieurs supports.

Les deux parties a) et b) du support selon l'invention sont réalisées de préférence en polystyrène ou en polycabonate ou toute matière plastique compatible avec la survie de cellules supérieures, et la membrane microporeuse, en nylon, en polypropylène ou en polycarbonate ou toute matière compatible avec l'attachement et la multiplication en monocouche de cellules supérieures. Cette membrane présente une porosité de 0,3 à 0,5 $\mu$m.

Les puits de culture ont par exemple une hauteur de 1 à 4 cm et un diamètre de 0,5 à 2 cm.

Après ensemencement des puits avec les cellules choisies et incubation à 37°C en milieu approprié jusqu'à obtention d'un tapis confluent sur les membranes microporeuses, le milieu de culture est vidé et remplacé par du milieu de maintenance, de manière à remplir la totalité du volume des puits, et, après expulsion de toute bulle d'air, les orifices supérieurs sont hermétiquement fermés par un film de plastique adhésif.

L'espace libre de la partie b) est rempli de milieu de maintenance additionné d'une substance gélifiante comme l'agarose (d'un degré de pureté compatible avec la survie des cellules supérieures). Ce milieu est ainsi capable de se solidifier dans les conditions de stockage et de transport du matériel, c'est-à-dire de +4°C à +10°C, et de redevenir liquide dans les conditions physiologiques précédant la mise en oeuvre du test (37°C).

Ainsi, quand le milieu de maintenance à l'agarose est gélifié, les puits de culture sont des enceintes totalement closes qui permettent le transport des tapis cellulaires prêts à l'emploi.

Différents types de cellules peuvent être mis en culture et conservés dans le support selon l'invention ; plus particulièrement, pour des tests de toxicité de produits cosmétiques, on utilise des fibroblastes humains (fibroblastes dérivés de prépuces ou lignées cellulaires établies) ou des fibroblastes de cornée de lapin (pour se rapprocher du modèle animal du test d'irritation de l'oeil de lapin connu sous le nom de test de Draize).

Le matériel selon l'invention, comprenant le support et les tapis cellulaires établis sur ses membranes et protégés par le milieu de maintenance gélifié, peut être stocké pendant quelques jours à basse température, de préférence entre 4°C et 10°C, et expédié aux utilisateurs dans les mêmes conditions peu contraignantes.

L'utilisation du matériel pour la mise en oeuvre de tests de toxicité ne nécessite aucune manipulation préalable ; une incubation de quelques heures à 37°C reliquéfie le milieu de maintenance et une incubation de 48 heures rétablit les cellules dans des conditions physiologiques.

Le matériel selon l'invention est ainsi particulièrement adapté à la mise en oeuvre du procédé décrit dans la demande de brevet FR 2 656 331 qui consiste à cultiver des cellules dans des chambres de culture traditionnelles présentant une membrane microporeuse permettant d'étudier la toxicité de préparations sur les cellules, par diffusion au travers de la membrane, quelle que soit la forme galénique de la préparation ou sa nature hydrosoluble ou liposoluble, les cellules restant dans leur milieu de culture physiologique par leur partie apicale. Le procédé était mis en oeuvre par manipulation des chambres de culture et transfert de celles-ci dans des cupules contenant les produits à tester.

L'avantage du matériel selon la présente invention est de permettre la mise en oeuvre des tests de toxicité sans aucune manipulation préalable. En effet, après incubation à 37°C jusqu'à ce que le milieu de maintenance gélifié soit redevenu liquide, il suffit de retirer la partie inférieure b) du support, de vider le milieu de maintenance et de le remplacer par la substance ou la composition à tester, soit en une dose unique , soit en plusieurs doses si le fond du support est compartimenté, et de le replacer en contact avec les cellules par simple réajustement avec la partie a). L'incubation est ensuite poursuivie à 37°C.

Après le temps de contact choisi, on mesure la viabilité des cellules attachées aux membranes microporeuses par un test colorimétrique classique.

Le matériel selon l'invention permet également de réaliser des tests plus sophistiqués et d'évaluer le métabolisme cellulaire en réaction aux produits testés.

Les exemples suivants illustrent l'invention sans en limiter la portée.

**EXEMPLE 1** : Mise en culture des cellules et étude de leur survie au cours du stockage et dans des conditions de test.

Des blocs cylindriques percés de trois puits ont été utilisés. La partie a) a un diamètre de 50 mm, une hauteur de 22 mm ; les puits ont un diamètre de 15 mm. La partie b) a un diamètre intérieur de 50 mm et extérieur de 60 mm et une hauteur de 12 mm. Avec ce modèle de bloc à 3 puits, chacune des trois chambres de culture est soumise aux mêmes conditions, pour donner une évaluation statistiquement valable des pourcentages de survie. Chacun des blocs est soumis aux conditions expérimentales choisies. Les cellules choisies sont des fibroblastes de cornée de lapin, en lignée établie.

Chaque chambre de culture est inoculée avec 500 000 cellules dans 1 ml de milieu (milieu MEM additionné de bicarbonate et de 10 % de sérum de veau foetal). L'espace libre de la partie inférieure (b) est rempli du même milieu.

Les cultures sont incubées à 37°C en atmosphère de $CO_2$ pendant 48 heures ce qui assure la formation d'une monocouche confluente sur la membrane microporeuse.

Après remplacement du milieu de croissance par du milieu de maintenance, additionné dans la partie inférieure b) de 1 % (P/V) d'agarose, les blocs sont placés en chambre froide à 4°C pendant différents temps, avec un minimum de 24 heures. A la sortie de la chambre froide, les blocs sont immédiatement incubés à 37°C pendant 48 heures et la viabilité des cellules est évaluée par un test colorimétrique au MTT, décrit par Mossman (J. Immunol. Methods, 1983, 55-63), et exprimée en pourcentage de cellules vivantes par rapport aux cultures parallèles maintenues à 37°C pendant le même temps.

On n'observe aucune perte de viabilité dans ces conditions.

Dans les mêmes conditions, après conservation à 4°C pendant 24 heures et incubation à 37°C pendant 48 heures, on étudie l'aptitude des cellules à subir un test de toxicité. Le compartiment inférieur est vidé du milieu à l'agarose et remplacé par un milieu à tester. Le produit à tester est de l'octocynol en solution dans du milieu de culture, à trois concentrations : 10, 50 et 100 µg/ml.

La viabilité cellulaire est mesurée comme décrit plus haut. On obtient les résultats suivants :

| Concentration d'octocynol en µg/ml | % de cellules survivantes après conservation à 4°C | % de cellules survivantes sur cultures fraîchement établies |
|---|---|---|
| 0 | 100 | 100 |
| 10 | 100 | 92 |
| 50 | 67,7 | 65 |
| 100 | 11 | 12 |

Les résultats ainsi obtenus sont les mêmes que ceux déterminés sur des cellules fraîchement établies et non conservées à 4°C en agarose.

**EXEMPLE 2** : Etude de cytotoxicité et détermination de la CL 50 du laurysulfate de sodium.

Des chambres de culture conformes au modèle décrit (une chambre témoin et 5 chambres destinées à recevoir des concentrations différentes du produit à étudier) sont ensemencées avec des fibroblastes humains préparés à partir de prépuces, à raison de 20 000 cellules par puits. Les chambres de cultures sont maintenues dans une étuve à 37°C.

24 heures plus tard, les cellules étant arrivées à confluence, le contenu de la partie b de chacune des chambres de culture est remplacé par de l'huile de paraffine, dans laquelle est dissous du laurysulfate de sodium, aux concentrations de 200 μg/ml, 100 μg/ml et 50 μg/ml ; de l'huile de paraffine seule est utilisée pour la chambre de culture servant de témoin (des essais préliminaires ont permis de s'assurer que l'huile de paraffine n'entraîne pas de mortalité de ce type cellulaire).

Après 24 heures de contact, la viabilité des cellules est estimée par le test colorimétrique au MTT et le pourcentage de cellules survivantes est calculé par rapport au témoin.

Les résultats suivants ont été obtenus:

| Concentration de SLS | % de cellules survivantes |
|---|---|
| 50 μg/ml | 100 |
| 100 μg/ml | 74,5 |
| 150 μg/ml | 6,0 |
| 200 μg/ml | 5,0 |

soit une CL 50 (concentration létale 50 %) calculée de 104,2 ± 13,6 μg/ml (moyenne ± écart type).

**EXEMPLE 3** : Evaluation de la toxicité parallèlement au pouvoir irritant oculaire d'un produit cosmétique.

L'essai est réalisé sur des fibroblastes humains, avec un lait démaquillant.

Dans les conditions d'utilisation décrites plus haut, les cellules étant arrivées à confluence sur les membranes microporeuses, une quantité choisie du produit cosmétique à étudier est mise dans le compartiment inférieur de 3 chambres de culture et recouverte par de l'huile de paraffine.

Les cultures sont replacées à l'étuve, respectivement pendant 30 minutes, 1 heure et 4 heures. Une chambre de culture témoin, ne contenant que de l'huile de paraffine, est laissée à l'étuve pendant 4 heures.

La viabilité des cellules est ensuite appréciée par le test colorimétrique au MTT.

Les résultats suivants ont été obtenus :

| Temps de contact avec le produit | % de cellules survivantes |
|---|---|
| 30 minutes | 72 |
| 1 heures | 57 |
| 4 heures | 0 |

Un indice de cytotoxicité a été calculé à partir de ces résultats, soit 2,25.

Des résultats semblables ont été obtenus avec 100 produits cosmétiques. Pour cet ensemble de résultats, l'indice de cytotoxicité et l'indice d'irritation oculaire maximum (méthode de DRAIZE) ont été déterminés, et ont permis de montrer qu'il existait une bonne corrélation (r = 0,85) entre ces deux paramètres.

Il est donc possible, à partir de l'indice de cytotoxicité, de déterminer un indice d'irritation oculaire théorique.

Dans le cas du produit cosmétique cité plus haut, cet indice est de 15,5, ce qui permet de considérer le produit comme légèrement irritant pour l'oeil.

La validité de ce résultat a été confirmée par la détermination de l'indice d'irritation oculaire maximum sur le lapin, par la méthode de DRAIZE. L'indice trouvé est de 13,3.

**EXEMPLE 4** : Description du support de cultures, prêt à l'envoi aux utilisateurs.

Divers prototypes conformes à la description générale ont été réalisés pendant la phase de mise au point, en particulier des blocs pleins, en matière plastique, dont les parties a et b s'emboîtent hermétiquement, de forme cylindrique ou parallèlépipédique.

Pour faciliter l'expédition aux utilisateurs et le stockage du matériel, des dispositifs allégés ont été réalisés, qui simplifient en outre leur manipulation ultérieure.

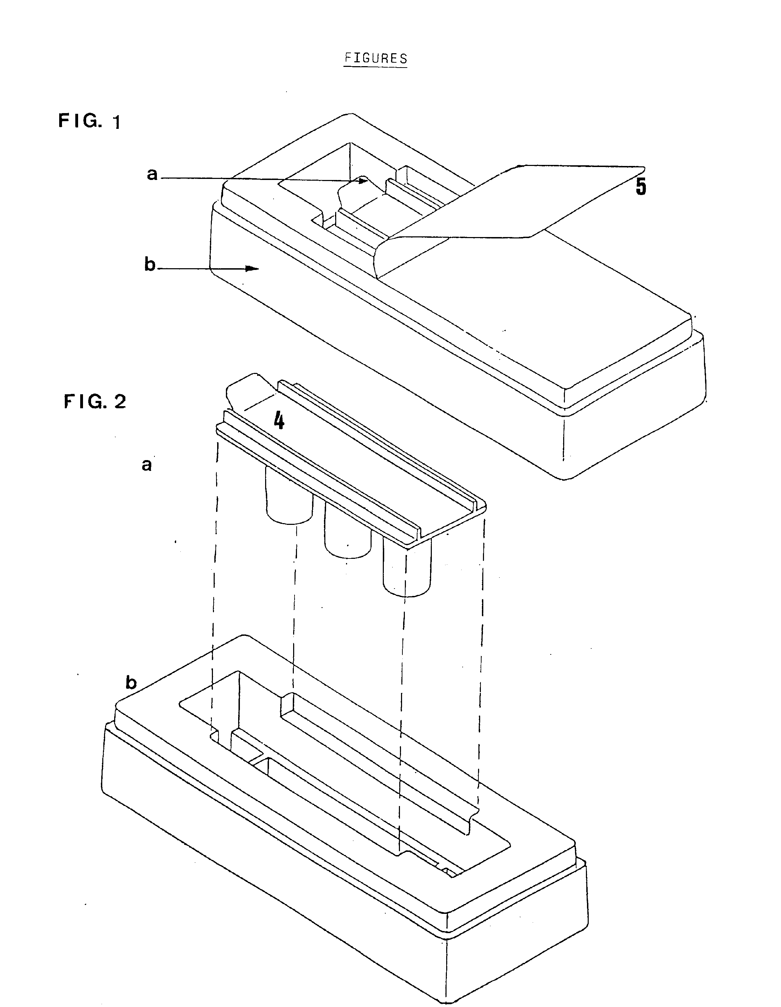

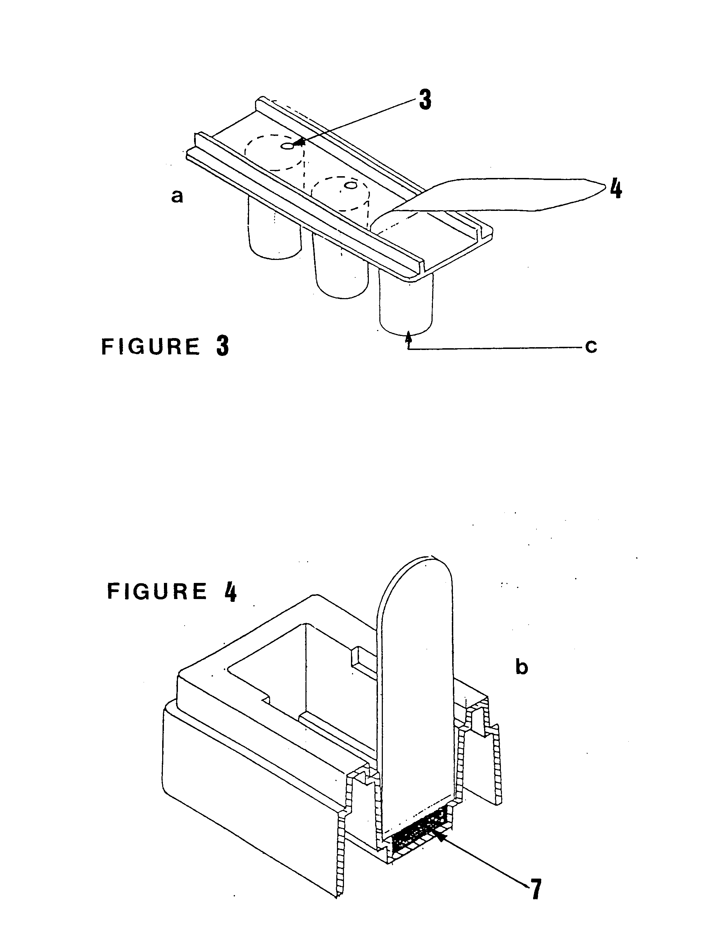

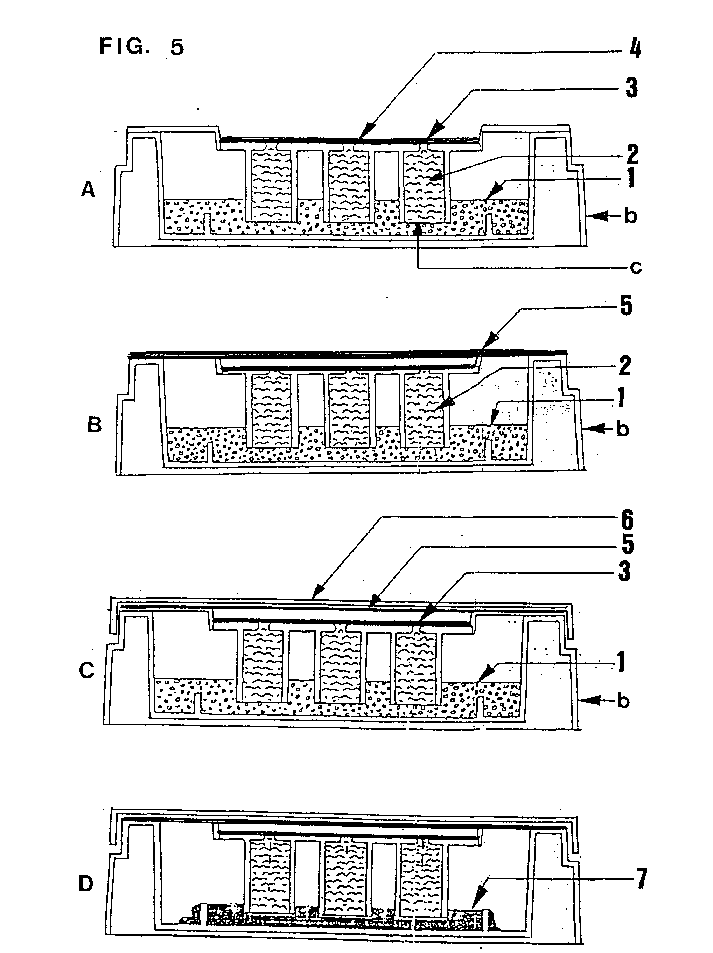

Les figures 1 à 5 représentent, de manière schématique, un mode de réalisation de ces dispositifs et leur mode d'utilisation. Ces représentations destinées à simplifier la description ne limitent les modes de réalisation possibles ni par la forme ni par le nombre des éléments constitutifs.

Légende des figures :

- Les mêmes chiffres ou lettres sont utilisés dans les 5 figures pour désigner les éléments suivants :
    a, b : 2 parties moulées du support, selon la revendication 1
    c : membrane microporeuse
    1 : milieu de maintenance gélifié, dans le support b
    2 : milieu de maintenance liquide, dans les puits de culture
    3 : orifice de remplissage des puits de culture
    4 : film de plastique adhésif obturant les puits de culture
    5 : film de plastique adhésif fermant hermétiquement l'ensemble du support a + b
    6 : couvercle protecteur
    7 : substance à tester;
- La figure 1 représente l'ensemble du support où les parties a et b sont emboîtées et fermées hermétiquement par un film de plastique adhésif (5).
- La figure 2 représente la partie a), à laquelle sont suspendus les puits de culture obturés sur la face supérieure par un film de plastique adhésif (4) et le retrait de cette partie a) qui permet l'échange de la partie b) avec un élément équivalent contenant la substance à tester (ou l'élimination du milieu de maintenance et son remplacement par la substance à tester).
- La figure 3 montre plus précisément les éléments constitutifs de la partie a) avec la position des orifices de remplissage (3) des puits de culture et de la membrane microporeuse.
- La figure 4 comporte une coupe transversale de la partie b), prête à l'utilisation, et qui montre la structure du dispositif favorisant l'étalement régulier de la substance à tester (7), si elle n'est pas liquide.
- La figure 5, représente l'ensemble des éléments et la sucession des manipulations du matériel prêt à l'emploi :
    . en 5A, le milieu additionné d'agarose (1) couvre le fond de la partie b) et est en contact avec la partie inférieure des membranes microporeuses (c) ; le milieu de maintenance liquide (2) remplit les puits de culture, des membranes microporeuses aux orifices de remplissage (3), ceux-ci étant obturés par un film de plastique adhésif (4).
    . en 5B, l'ensemble du dispositif a+b est fermé hermétiquement par un second film de plastique adhésif (5) et celui-ci est protégé par un couvercle (6) (en 5c).
    . en 5D, dans la partie b), le milieu de maintenance est remplacé par la substance à tester (7).

**EXEMPLE 5** : Mise en oeuvre du test par l'utilisateur

Le matériel prêt à l'emploi est expédié sous emballage de polystyrène contenant de la glace.

Il est livré avec les tapis de cellules confluentes (le choix des cellules est dicté par la nature du test à effectuer) et ne nécessite pas d'autre manipulation qu'une incubation de 48 heures à 37°C, pour reliquéfier le milieu de maintenance et rétablir les cellules dans des conditions physiologiques normales.

La mise en oeuvre du test comprend :
- l'élimination de l'élément b) avec le milieu de maintenance et son remplacement par un élément identique contenant la substance à tester ;
- une incubation à 37°C pendant la durée voulue ;
- le remplacement de l'élément b) par un élément identique contenant le réactif révélateur de la viabilité cellulaire (rouge neutre ou MTT) ; à ce stade, les puits sont ouverts pour la première fois, vidés de leur contenu, remplis d'un volume déterminé du même réactif et incubés à 37°C pendant 3 heures.
- la détermination de la quantité de réactif coloré absorbé par les cellules, par mise en solution de celui-ci dans du solvant (isopropanol ou acide acétique/éthanol) directement dans les puits préalablement vidés ; la mesure de la coloration est effectuée au spectrophotomètre et corrélée à celle de puits témoins ; la viabilité est exprimée en pourcentage par rapport aux témoins.

## Revendications

1. Support de cultures cellulaires pour matériel prêt à l'emploi caractérisé en ce qu'il se présente sous forme d'un moulage en matière plastique en deux parties ajustées :
   - une partie a) constituée d'un monobloc dans lequel sont creusés des puits cylindriques destinés à servir de chambres de cultures, leur partie inférieure étant constituée d'une membrane microporeuse apte à supporter la multiplication et le maintien de cultures cellulaires et leur partie supérieure étant obturée à l'exception des orifices de remplissage qui seront fermés ultérieurement par un film de plastique adhésif ;
   - une partie b) adaptée sous la partie a) pour la fermer hermétiquement tout en maintenant un espace libre destiné à mettre les membranes microporeuses en contact avec du milieu de culture ou de maintenance et, ultérieurement, avec la substance à tester, ledit espace libre pouvant être commun à quelques puits de culture ou compartimenté pour isoler chaque puits de culture ;
   - et, les deux parties a) et b) étant emboîtées, l'ensemble est de préférence fermé par un second film de plastique adhésif et recouvert d'un couvercle protecteur permettant la superposition de plusieurs supports.

2. Support de cultures cellulaires selon la revendication 1, caractérisé en ce qu'il est fabriqué dans une matière choisie parmi le polystyrène, le polycarbonate ou toute matière plastique compatible avec la survie de cellules supérieures.

3. Support de cultures cellulaires selon la revendication 1 ou 2, caractérisé en ce que les puits cylindriques ont une hauteur d'environ 1 à 4 cm et un diamètre d'environ 0,5 à 2 cm.

4. Support de cultures cellulaires selon l'une quelconque des revendiations 1 à 3, caractérisé en ce que les membranes microporeuses sont fabriquées en une matière choisie parmi le nylon, le polycarbonate, le polypropylène ou toute matière compatible avec l'attachement et la multiplication d'une monocouche de cellules supérieures.

5. Support de cultures cellulaires selon l'une quelconque des revendications 1 à 4, caractérisé en ce que les membranes microporeuses ont une porosité de 0,3 à 0,5 $\mu$m.

6. Matériel prêt à l'emploi pour la réalisation d'études de toxicologie et de pharmacologie cellulaires, caractérisé en ce qu'il comprend un support de cultures cellulaires selon l'une quelconque des revendications 1 à 5, un tapis cellulaire confluent établi sur les membranes microporeuses de chaque puits et, dans les espaces supérieur et inférieur entourant les membranes et les tapis cellulaires, du milieu de maintenance, additionné dans la partie b) d'une substance gélifiante qui permet au milieu de se présenter à l'état gélifié à basse température et à l'état liquide à température physiologique.

7. Matériel prêt à l'emploi selon la revendication 6, caratérisé en ce que le tapis cellulaire confluent est établi à partir d'une suspension de cellules, en cultures primaires ou en lignées, choisies parmi les divers types de cellules supérieures appropriées pour des études de cytotoxicité.

8. Matériel selon la revendication 6 caractérisé en ce que la substance gélifiante est l'agarose, de qualité purifiée pour cellules supérieures.

9. Matériel prêt à l'emploi selon l'une quelconque des revendications 6 à 8, caractérisé en ce qu'il supporte le stockage et le transport à une température comprise entre 4°C et 10°C.

10. Méthode d'étude de toxicologie et de pharmacologie cellulaire d'une substance ou d'une composition, caractérisée en ce qu'elle comprend l'incubation du matériel selon l'une quelconque des revendications 6 à 9, à 37°C pendant 48 heures sans autres manipulation, puis la mise en place de la substance ou de la composition dans la partie b) du support selon la revendication 1, en contact avec les cultures cellulaires au travers des membranes microporeuses et, après un temps de contact choisi, la détermination du pourcentage de cellules survivantes.

FIGURES

**FIG. 1**

a

b

5

**FIG. 2**

4

a

b

FIGURE 3

FIGURE 4

FIG. 5

**Office européen des brevets**

**RAPPORT DE RECHERCHE EUROPEENNE**

Numero de la demande

EP     92 40 2616

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X<br>Y | EP-A-0 359 249 (W.R. GRACE & CO)<br>* revendication 44; figures 15,18 *<br>* colonne 13, ligne 30 - colonne 14, ligne 20 * | 1-5,7,9<br>1,6,8,10 | C12M1/34<br>C12Q1/18<br>C12M3/06 |
| Y<br>A | EP-A-0 308 129 (ALCAN INTERNATIONAL LTD)<br>* revendications; figures *<br>* page 3, ligne 47 - page 4, ligne 7 *<br>* page 4, ligne 19 - ligne 27 * | 6,8,10<br>1-5 | |
| A | DE-A-2 157 150 (MILES LABORATORIES INC)<br>* revendications; figures * | 1 | |
| Y | FR-A-2 656 331 (BIOGIR)<br>* le document en entier * | 1,6,8,10 | |
| Y | WO-A-9 013 624 (HUMAN MEDICAL LABORATORIES INC)<br>* revendications; figures 4,5 *<br>* page 10, alinéa 2 *<br>* page 11 *<br>* page 12, ligne 1 - ligne 3 * | 1,6,8,10 | |
| Y | US-A-2 761 813 (A. GOETZ)<br>* revendications 1,2,6; figures *<br>* colonne 8, ligne 38 - ligne 56 *<br>* colonne 7, ligne 37 - ligne 67 * | 6,8,10 | **DOMAINES TECHNIQUES RECHERCHES (Int. Cl.5)**<br><br>C12M<br>C12Q |
| A | EP-A-0 408 940 (MILLIPORE CORPORATION)<br>* revendications; figures *<br><br>----- | 1 | |

Le présent rapport a été établi pour toutes les revendications

| Lieu de la recherche | Date d'achèvement de la recherche | Examinateur |
|---|---|---|
| LA HAYE | 21 JANVIER 1993 | COUCKE A.O.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)